# EUROPEAN PATENT SPECIFICATION

(11) **EP 3 015 066 B1**
(45) Date of publication and mention of the grant of the patent: **14.02.2024**
(21) Application number: 15191624.4
(22) Date of filing: 27.10.2015
(51) Int. Cl.: A61B 5/145

(54) **SENSOR INSERTION METHOD, ANALYSIS METHOD, AND SENSOR INSERTION DEVICE**
SENSOREINSETZVERFAHREN, ANALYSEVERFAHREN UND SENSOREINSETZVORRICHTUNG
PROCÉDÉ D'INSERTION DE CAPTEUR, PROCÉDÉ D'ANALYSE ET DISPOSITIF D'INSERTION DE DÉTECTEUR

(30) Priority: 31.10.2014 JP 2014222220; 29.09.2015 JP 2015191299
(43) Date of publication of application: 04.05.2016
(73) Proprietor: ARKRAY, Inc., Minami-ku Kyoto-shi Kyoto 601-8045 (JP)
(72) Inventor: GO, Gai, Kyoto, 602-0008 (JP); Yamamoto, Akihiro, Kyoto, 602-0008 (JP)
(74) Representative: Hoffmann Eitle

(56) References cited:
- EP-A1- 2 517 625
- EP-A1- 2 754 392
- EP-A2- 2 215 964
- EP-B1- 2 215 964
- WO-A1-2007/097754
- WO-A1-2013/136968
- US-A1- 2007 078 322
- US-A1- 2012 184 909

## Description

### BACKGROUND OF THE INVENTION

### 1. Field of the Invention:

The present invention relates to a sensor insertion method, an analysis method, and a sensor insertion device for inserting a sensor into a user's body.

### 2. Description of Related Art:

For healthcare for patients, analysis methods are widely used that collect a body fluid such as blood and perform an analysis of a specific component using the collected fluid as a sample. Examples of these analysis methods include self-monitoring of blood glucose (SMBG) in which glucose is taken as a specific component. In this sort of analysis methods, for example, an analysis is performed at timings of daily activities such as before eating or after eating. Accordingly, the analysis results are obtained as data that is discrete in the time axis.

Meanwhile, in recent years, there are proposed methods that continuously acquire analysis results by leaving a sensor or the like in a user's body. Examples of these analysis methods include continuous glucose measurement system (CGMS) (see Japanese Patent No. 4778100, for example). In this analysis method, a sensor using a principle based on electrochemistry is inserted into a patient's body. If the analysis using this sensor is continuously or more frequently performed, the user's health condition can be more precisely known.

Also in WO 2007/097754 A1, US 2012/184909 A1, EP2215964 A2 and EP2754392 A2 similar analysis methods and devices are described.

In order to realize the analysis using this sensor, it is common to use a sensor control unit that is electrically connected to the sensor. In the method disclosed in Japanese Patent No. 4778100, after the sensor is inserted into an insertion target portion, which is part of the user's body, a doctor, a nurse, or the user has to connect the sensor control unit to the sensor inserted into the insertion target portion. The sensor is in the shape of, for example, a needle, and, thus, the connecting operation is not easy. Furthermore, the insertion target portion may be a portion that cannot be easily seen such as the abdominal region near the back. Accordingly, there is a problem that it is difficult to connect the sensor and the sensor control unit or that, at the time of connection, the sensor is pressed to move inside the body to cause pain, for example.

### SUMMARY OF THE INVENTION

The present invention has been proposed under the above-described circumstances, and an object of the invention is to provide a sensor insertion method, an analysis method, and a sensor insertion device, capable of more properly connecting a sensor and a sensor control unit.

The present invention is defined by the appended independent claims 1 and 5. The respective dependent claims describe optional features and preferred embodiments.

According to a first aspect of the present invention, there is provided a sensor insertion method for inserting a sensor into a user's body. The method includes: an attaching step of attaching the sensor to a unit base; a unit fixing step of regulating the relative position between a sensor control unit, which is in a sensor insertion allowing state, and an inserting unit that contains the sensor, and further of fixing the sensor control unit and the inserting unit to an insertion target portion of the user, wherein the sensor control unit being fixed inside the inserting unit; a sensor inserting step of causing the inserting unit to insert the sensor, which is attached to the unit base, into the insertion target portion; a fixing step of fixing unit base and thereby also the sensor, which is attached to the unit base, in a state that the sensor is inserted into the insertion target portion of the user; and a sensor connecting step of shifting the sensor control unit from the sensor insertion allowing state to a sensor connecting state, thereby establishing electrical conduction between the sensor in the insertion target portion and the sensor control unit, wherein in the sensor connecting step, the sensor and the sensor control unit are electrically connected to each other by rotating of the sensor control unit, wherein the sensor control unit rotatably engages with the unit base by moving of the inserting unit along the insertion target portion.

In a preferable embodiment, in the unit fixing step, after the relative position between the sensor control unit and the inserting unit is regulated, the sensor control unit and the inserting unit are fixed to the insertion target portion.

In a preferable embodiment, the sensor insertion method further includes a unit releasing step of releasing the inserting unit from the insertion target portion after the sensor connecting step.

According to a second aspect of the present invention, there is provided an analysis method including an analysis step of performing an analysis using the sensor after the sensor insertion method of the first aspect is performed.

According to a third aspect of the present invention, there is provided a sensor insertion device for inserting a sensor for analysis into a user's body. The device includes: an inserting unit that inserts the sensor into an insertion target portion of the user; a unit base that the sensor is attached and which is installed inside the inserting unit; and a sensor control unit regulated in relative position with respect to the inserting unit, being fixed inside the inserting unit and configured to selectively take a sensor insertion allowing state allowing the insert of the sensor while the inserting unit is fixed to the insertion target portion, and a sensor connecting state in which the sensor control unit is electrically connected to the sensor inserted into the insertion target portion, wherein the inserting unit causes the unit base and the sensor attached to the unit base to move, inserts the sensor into the insertion target portion, and fixes the unit base on the insertion target portion, and wherein the unit base fixes the sensor in a state that the sensor is inserted into the insertion target portion of the user, wherein the sensor control unit is configured to establish an electrical conduction between the sensor and the sensor control unit by: moving a part or the whole of the sensor control unit, or rotation of the sensor control unit, wherein the sensor control unit is configured to rotatably engage with the unit base by moving of the inserting unit along the insertion target portion.

In a preferable embodiment, the sensor control unit is shifted from the sensor insertion allowing state to the sensor connecting state when the sensor control unit is moved in a direction along a surface of the insertion target portion.

According to embodiments of the present invention, when the unit fixing step is completed, the relative position between the sensor inserting unit and the sensor control unit in the sensor insertion allowing state is regulated. Subsequently, after the sensor inserting step is ended, the sensor control unit is shifted from the sensor insertion allowing state to the sensor connecting state, and thus, the electirical connection between the sensor and the sensor control unit is completed. Accordingly, it is not necessary for a user to connect the sensor control unit e.g. by trial and error to the sensor inserted. Hence, the sensor and the sensor control unit can be more properly connected to each other.

Other features and advantages of the present invention will become more apparent from the detailed description given below with reference to the attached drawings.

### BRIEF DESCRIPTION OF THE DRAWINGS

Fig. 1 is a perspective view showing a unit fixing step in a sensor insertion method according to a first embodiment of the present invention.
Fig. 2 is a perspective view showing the unit fixing step in the sensor insertion method according to the first embodiment of the present invention.
Fig. 3 is a perspective view showing the unit fixing step in the sensor insertion method according to the first embodiment of the present invention.
Fig. 4 is a perspective view showing a sensor inserting step in the sensor insertion method according to the first embodiment of the present invention.
Fig. 5 is a perspective view showing a state in which the sensor inserting step is completed in the sensor insertion method according to the first embodiment of the present invention.
Fig. 6 is a perspective view showing a sensor connecting step in the sensor insertion method according to the first embodiment of the present invention.
Fig. 7 is a system configuration diagram showing an analysis system in the sensor insertion method according to the present invention.
Fig. 8 is a perspective view showing a unit fixing step in a sensor insertion method according to a second embodiment of the present invention.
Fig. 9 is a perspective view showing the unit fixing step in the sensor insertion method according to the second embodiment of the present invention.
Fig. 10 is a perspective view showing a unit fixing step in a sensor insertion method according to a third embodiment of the present invention.
Fig. 11 is a perspective view showing the unit fixing step in the sensor insertion method according to the third embodiment of the present invention.
Fig. 12 is a perspective view showing a sensor inserting step in the sensor insertion method according to the third embodiment of the present invention.
Fig. 13 is a perspective view showing a sensor connecting step in the sensor insertion method according to the third embodiment of the present invention.
Fig. 14 is a perspective view showing a unit fixing step in a sensor insertion method according to a fourth embodiment of the present invention.
Fig. 15 is a perspective view showing the unit fixing step in the sensor insertion method according to the fourth embodiment of the present invention.
Fig. 16 is a perspective view showing the unit fixing step in the sensor insertion method according to the fourth embodiment of the present invention.
Fig. 17 is a cross-sectional view showing a sensor insertion device according to a fifth embodiment of the present invention.
Fig. 18 is a cross-sectional view showing a unit fixing step in a sensor insertion method according to the fifth embodiment of the present invention.
Fig. 19 is a cross-sectional view showing a sensor inserting step in the sensor insertion method according to the fifth embodiment of the present invention.
Fig. 20 is a cross-sectional view showing the sensor inserting step in the sensor insertion method according to the fifth embodiment of the present invention.
Fig. 21 is a cross-sectional view showing a sensor connecting step in the sensor insertion method according to the fifth embodiment of the present invention.
Fig. 22 is a cross-sectional view showing a state in which the sensor connecting step is completed in the sensor insertion method according to the fifth embodiment of the present invention.
Fig. 23 is a perspective view showing a unit fixing step in a sensor insertion method according to a sixth embodiment of the present invention.
Fig. 24 is a perspective view showing a sensor inserting step in the sensor insertion method according to the sixth embodiment of the present invention.
Fig. 25 is a perspective view showing a sensor connecting step in the sensor insertion method according to the sixth embodiment of the present invention.
Fig. 26 is a cross-sectional view showing a sensor insertion device according to a seventh embodiment of the present invention.
Fig. 27 is a cross-sectional view showing a sensor inserting step in a sensor insertion method according to the seventh embodiment of the present invention.
Fig. 28 is a cross-sectional view showing the sensor inserting step in the sensor insertion method according to the seventh embodiment of the present invention.
Fig. 29 is a cross-sectional view showing a step following the sensor inserting step in the sensor insertion method according to the seventh embodiment of the present invention.
Fig. 30 is a cross-sectional view showing a sensor connecting step in the sensor insertion method according to the seventh embodiment of the present invention.

### DESCRIPTION OF THE PREFERRED EMBODIMENTS

Preferred embodiments of the present invention will be specifically described with reference to the drawings.

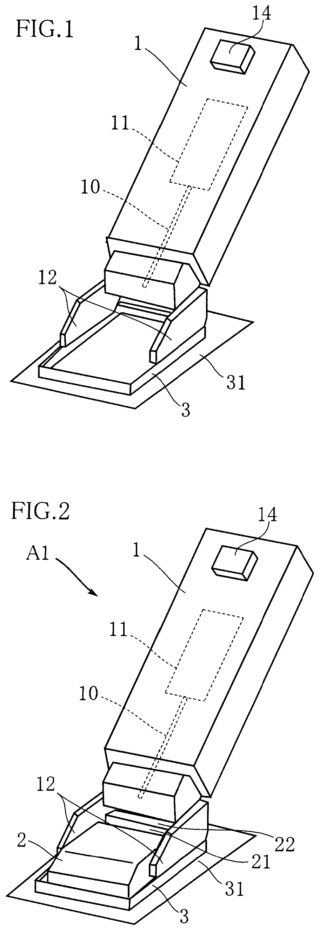

Below, a sensor insertion method, an analysis method, and a sensor insertion device according to the first embodiment of the present invention will be described with reference to Figs. 1 to 6. In this embodiment, a sensor insertion method and an analysis method using a system configured as CGMS in which glucose is taken as a specific component and a sensor insertion device used in the sensor insertion method and the analysis method will be described, but the sensor insertion method, the analysis method, and the sensor insertion device according to the present disclosure are not limited to this application. There is no limitation on the analysis method according to the present disclosure, as long as it is a method that performs an analysis of a specific component by inserting a sensor into a body. The analysis maybe continuously performed, or may be periodically performed. The sensor insertion device may have various configurations according to sensors that are used. In the following embodiment, a case will be described as an example in which anelectrochemical sensor using a principle based on electrochemistry is used as a sensor, but the sensor according to the present disclosure may be sensors other than the electrochemical sensor.

Figs. 1 to 3 show a unit fixing step in this embodiment. First, as shown in Fig. 1, a sensor inserting unit 1, a unit base 3, and a securing tape 31 are prepared. The sensor inserting unit 1 contains a sensor 10, and is a unit for inserting the sensor 10 into an insertion target portion of a user. The sensor 10 in a state of being inserted into the user's body realizes a continuous or periodical analysis.

There is no particular limitation on the configuration of the sensor 10, but, in this embodiment, the sensor is a device having a base member in the shape of a needle or an extremely thin band made of a material that does not influence human bodies, such as polyimide resin. In order to realize an analysis based on an electrochemical principle, the base member of the sensor 10 is provided, for example, at a tip thereof, with an analysis electrode (not shown) . The analysis electrode is made of, for example, part of a wiring pattern formed on the base member. The wiring pattern is made of a metal selected as appropriate, and is formed by a method such as printing or plating. Furthermore, the sensor 10 has an electrode pad 101, which will be described later. The electrode pad 101 is to be electrically connected to a sensor control unit 2, which will be described later, and, in this embodiment, is formed on the base end side of the sensor 10. In this embodiment, the sensor 10 is of a disposable type, that is, is intended to be disposed of after being used for a single time of sensor insertion and analysis.

The sensor inserting unit 1 has an inserting portion 11 for inserting the sensor 10. Before use, the inserting portion 11 may hold as appropriate the sensor 10 contained in the sensor inserting unit 1. The inserting portion 11 has a function of inserting the sensor 10 into the user's body in response to a predetermined operation of the user. In order to realize this function, the inserting portion 11 is provided with a biasing means such as a spring (not shown) for applying a biasing force with which the sensor 10 can be inserted into the body. Furthermore, the inserting portion 11 has a function of releasing the sensor 10 from the sensor inserting unit 1 after the sensor 10 is inserted into the body. This releasing is realized by canceling the holding (e.g., pinching) of the sensor 10, cutting part of the sensor 10, or the like, and the inserting portion 11 is provided as appropriate with a releasing means for realizing this function. If the user presses down an operation portion 14, for example, the inserting portion 11 performs the above-described inserting operation and releasing operation.

There is no particular limitation on the shape and the material of the sensor inserting unit 1, and any configuration is possible as long as the sensor 10 can be properly inserted into the body. In this embodiment, the sensor inserting unit 1 is as a whole in the shape of an elongated rectangular column. The sensor inserting unit 1 has as appropriate a casing made of resin or the like, a rigidity-supporting portion made of metal, and the like. Furthermore, the sensor inserting unit 1 may be formed in one piece, or may have a configuration that can be separated into a plurality of portions, for the convenience of the user. In this embodiment, the sensor inserting unit 1 has a shape that allows the sensor 10 to be inserted obliquely with respect to the surface of the insertion target portion.

Furthermore, in this embodiment, the sensor inserting unit 1 has an engagement portion 12. The engagement portion 12 is to be engaged with the unit base 3, and is used to fix the sensor inserting unit 1 to the insertion target portion. The engagement between the engagement portion 12 and the unit base 3 may be realized, for example, by causing a catch (it is not shown) formed on the engagement portion 12 to be engaged with a recess or a hole (they are not shown) formed on the unit base 3, but there is no limitation to such a configuration. In this embodiment, two engagement portions 12 are provided such that a region in which the sensor control unit 2 is to be disposed is held between the engagement portions 12. In this embodiment, the sensor inserting unit 1 is not of a disposable type, that is, is intended to be repeatedly used for a plurality of times of sensor insertion.

The unit base 3 is used to fix the sensor inserting unit 1 to the insertion target portion of the user. Furthermore, in this embodiment, the unit base 3 is used to fix the sensor control unit 2 to the insertion target portion. That is to say, it can be assured that, if the thus configured unit base 3 is used, a configuration is realized in which the relative position between the sensor inserting unit 1 and the sensor control unit 2 is regulated via the unit base 3.

The unit base 3 is secured to the insertion target portion, and, in this embodiment, is secured by the securing tape 31. The securing tape 31 is an example of a means for securing the unit base 3 to the insertion target portion, and may have any configuration as long as it can exhibit a securing force with which the unit base 3, to which the sensor inserting unit 1 and the sensor control unit 2 are fixed, can be properly fixed to the insertion target portion. Note that the means for securing the unit base 3 to the insertion target portion is not limited to the securing tape 31, and, for example, may be an adhesive agent that is applied to the back face of the unit base 3. Furthermore, although the configuration using the unit base 3 is used as a preferable configuration for realizing the sensor insertion method and the analysis method of this embodiment, the sensor insertion method and the analysis method according to the present disclosure are not limited thereto, and, for example, the sensor inserting unit 1 and the sensor control unit 2 may be fixed to the insertion target portion without using the unit base 3. In this embodiment, the unit base 3 is of a disposable type, that is, is intended to be disposed of after being used for a single time of sensor insertion and analysis.

As shown in Fig. 1, if the engagement portions 12 of the sensor inserting unit 1 are engaged with the unit base 3, the sensor inserting unit 1 and the unit base 3 are fixed to each other. Note that, before or after the fixing, the securing tape 31 is attached to the unit base 3.

Next, as shown in Fig. 2, the sensor control unit 2 is attached to the unit base 3. The attachment of the sensor control unit 2 is realized, for example, by causing part of the sensor control unit 2 to be engaged with a groove or a recess (they are not shown) formed on the unit base 3. In this embodiment, if the sensor control unit 2 is engaged with the unit base 3, the relative position of the whole of the sensor control unit 2 is fixed with respect to the sensor inserting unit 1, and the whole of the sensor control unit 2 is not moved with respect to the sensor inserting unit 1. This state is an example of a state in which the relative position between the sensor inserting unit 1 and the sensor control unit 2 is regulated.

In this embodiment, the sensor control unit 2 has a through portion 21 and a sliding portion 22. The through portion 21 is a space through which the sensor 10 inserted from the sensor inserting unit 1 is allowed to pass toward the insertion target portion. Examples of specific configurations of the through portion 21 include a hole that extends in the upper-lower direction in the drawings through the sensor control unit 2.

The sliding portion 22 is a portion that can be relatively moved with respect to the other portions of the sensor control unit 2. In this embodiment, the slidingportion 22 is positioned on the rear side in the sensor control unit 2 in the drawings, and is slidable in the front-rear direction in the drawings along the direction in which the securing tape 31 extends . When the sliding portion 22 is withdrawn rearward in the drawings, the through portion 21 is formed. In this embodiment, the state in which the sliding portion 22 is withdrawn rearward to form the through portion 21 corresponds to a sensor insertionallowing state in the present disclosure. In this embodiment, the sensor control unit 2 is not of a disposable type, that is, is intended to be repeatedly used for a plurality of times of sensor insertion.

When the attachment of the sensor control unit 2 to the unit base 3 is completed as shown in Fig. 2, a sensor insertion device A1 of this embodiment is completed. The sensor insertion device A1 includes the sensor inserting unit 1, the sensor control unit 2, and the unit base 3 described above.

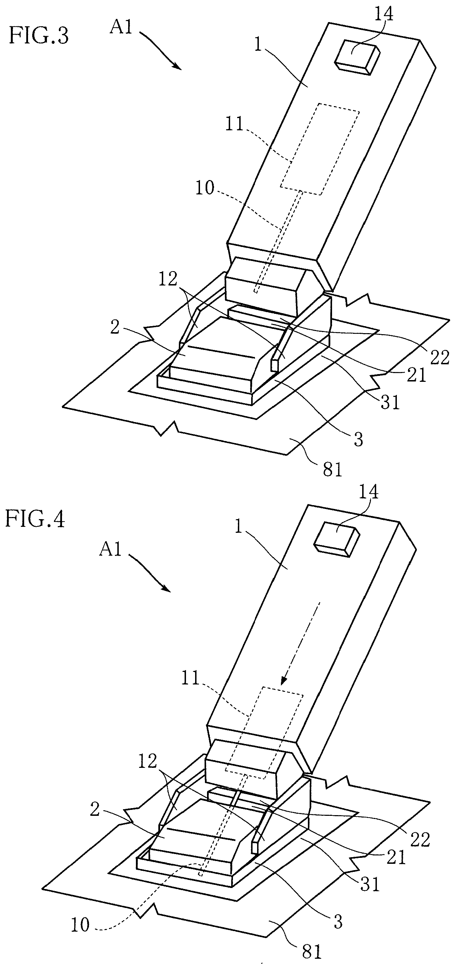

Next, as shown in Fig. 3, the securing tape 31 is attached to an insertion target portion 81, which is part of the user's body, so that the unit base 3 is secured to the insertion target portion 81. Accordingly, the sensor inserting unit 1 and the sensor control unit 2 are fixed to the insertion target portion 81 via the securing tape 31 and the unit base 3. At that time, the sensor control unit 2 is fixed to the insertion target portion 81 while maintaining the sensor insertion allowing state, and the relative position between the sensor inserting unit 1 and the sensor control unit 2 is regulated.

Fig. 4 shows a sensor inserting step in this embodiment. In this step, if the user performs a predetermined operation such as pressing the operation portion 14 formed on the sensor inserting unit 1, the sensor 10 is inserted by the inserting portion 11 of the sensor inserting unit 1 through the through portion 21 of the sensor control unit 2 into the insertion target portion 81. At that time, a desired portion of the sensor 10 is inserted into the insertion target portion 81, by a biasing force by the above-described biasing means.

In this embodiment, the sensor 10 is made of a flexible material such as polyimide resin, and, thus, the sensor 10 is inserted together with a puncture assisting member (not shown) by the inserting portion 11 into the insertion target portion 81. The puncture assisting member is for assisting puncture of the sensor 10 into the insertion target portion 81, and, is, for example, a long and thin member that is relatively rigid and is made of metal or the like in a needle-like shape, a shape with a U-shaped cross-section, an extremely thin band- like shape, or the like. After the sensor 10 is inserted, the inserting portion 11 exhibits the function of removing the puncture assisting member from the insertion target portion 81 so as to be separated from the sensor 10. In parallel to the removal of the puncture assisting member, the inserting portion 11 releases the sensor 10. This releasing may be realized by canceling the holding (e.g. , pinching) of the sensor 10, cutting part of the sensor 10, or the like. With this processing, the inserted sensor 10 is left in the insertion target portion 81, and the sensor inserting step in the present disclosure is completed. Note that the puncture assisting member is of a disposable type, that is, is intended to be disposed of after being used for a single time of sensor insertion and analysis. If the sensor 10 is rigid enough to realize the sensor inserting step with the sensor 10 alone, a configuration without using the puncture assisting member is also possible.

If the sensor inserting unit 1 (the inserting portion 11) is fixed to the insertion target portion 81 in the orientation shown in the drawing, the sensor 10 is inserted in an orientation that is inclined with respect to the surface of the insertion target portion 81. Furthermore, if the sensor 10 is relatively rigid, the sensor inserting step may be performed without using the above-described puncture assisting member.

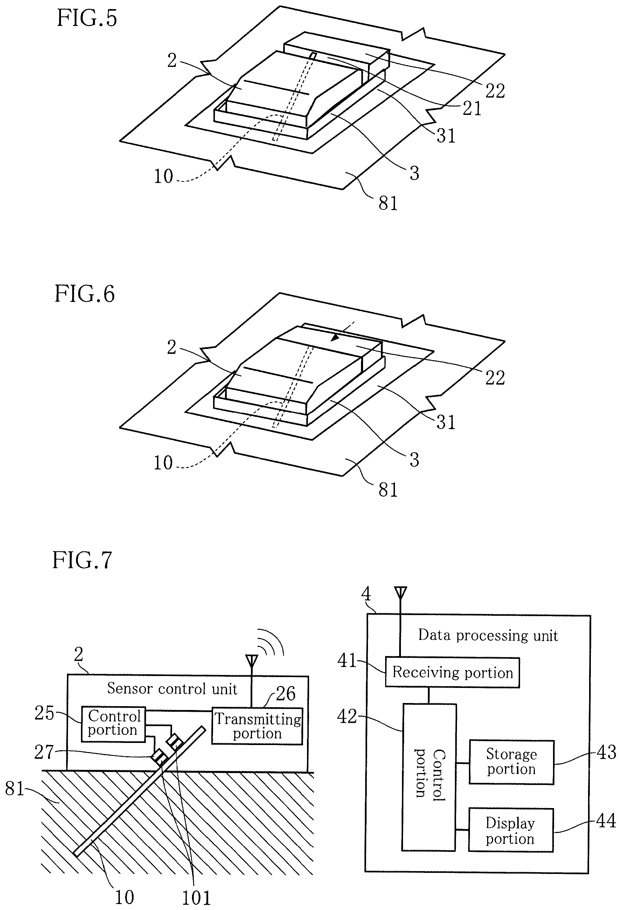

Next, as shown in Fig. 5, the sensor inserting unit 1 is released from the unit base 3. Accordingly, the unit base 3 and the sensor control unit 2 are fixed via the securing tape 31 to the insertion target portion 81, and the state is realized in which the sensor 10 is inserted.

Next, as shown in Fig. 6, a sensor connecting step is performed. In this embodiment, the sliding portion 22 of the sensor control unit 2 is slid forward in the drawing to close the through portion 21, so that the sensor 10 is held by the sensor control unit 2. This sliding operation is used to electrically connect the sensor control unit 2 and the sensor 10. Accordingly, the sensor control unit 2 is in the sensor connecting state in the present disclosure. Note that the sliding processing of the sliding portion 22 shown in the drawing is an example of processing that moves the sliding portion 22 in a direction in which the securing tape 31 extends, that is, in a direction along the surface of the insertion target portion 81. This movement direction intersects the sensor 10 inserted into the insertion target portion 81.

Fig. 7 shows the sensor control unit 2 in a state where the sensor connecting step is completed. In the example shown in the drawing, the sensor 10 is provided with a plurality of electrode pads 101. There is no particular limitation on the shape, the arrangement, or the number of electrode pads 101. Furthermore, the sensor control unit 2 is provided with a plurality of connectors 27 that are to be connected to the plurality of electrode pads 101. In this embodiment, if the sliding portion 22 is slid, the plurality of electrode pads 101 and the plurality of connectors 27 are brought into contact with each other. Accordingly, the sensor 10 and the sensor control unit 2 are electrically connected to each other, and the sensor connecting state is realized.

In the example shown in the drawing, the sensor control unit 2 has a control portion 25 and a transmitting portion 26. The control portion 25 realizes an electrochemical analysis using the sensor 10, and sends the transmitting portion 26 an electrical signal indicating an analysis result and a command for transmission control. The transmitting portion 26 transmits an electrical signal regarding an analysis result, to a unit outside the sensor control unit 2. In this embodiment, the transmitting portion 26 can transmit signals using wireless communication.

A data processing unit 4 is a unit that receives an electrical signal regarding an analysis result transmitted from the sensor control unit 2, and, in the example shown in the drawing, includes a receiving portion 41, a control portion 42, a storage portion 43, and a display portion 44. The receiving portion 41 receives an electrical signal from the sensor control unit 2, and, in this embodiment, can receive signals using wireless communication.

The control portion 42 performs data processing, for example, by executing a predetermined program on the electrical signal regarding an analysis result received by the receiving portion 41. If necessary, the control portion 42 stores the analysis result data in the storage portion 43. The storage portion 43 is configured by, for example, a semiconductor memory device, and the control portion 42 can write and read analysis result data to and from the storage portion 43. The display portion44 is, for example, a liquid crystal display, and displays as appropriate current data such as current date and time, as well as a receiving state by the receiving portion 41, a processing state by the control portion 42, a storing state by the storage portion 43, freely selected analysis results, and the like.

The data processing unit 4 may perform data exchange only with one sensor control unit 2, or may perform data exchange with a plurality of sensor control units 2. The sensor control unit 2 and the data processing unit 4 having such a configuration form an analysis system for performing the analysis method in this embodiment.

After the analysis system shown in Fig. 7 is constructed, an analysis step using the sensor 10 is continuously or periodically executed. At the execution of the analysis step, the sensor control unit 2 may control execution timings based on a time measuring means included in the sensor control unit 2, or the sensor control unit 2 may perform an analysis in response to a request from the data processing unit 4 based on a time measuring means included in the data processing unit 4. In the case of continuously performing an analysis, an analysis using the sensor 10 is performed substantially on a steady basis, and analysis results thereof are transmitted as electrical signals from the transmitting portion 26 of the sensor control unit 2 to the receiving portion 41 of the data processing unit 4. The transmission from the transmitting portion 26 to the receiving portion 41 may be performed substantially on a steady basis, or analysis results in a predetermined period may be at a time transmitted at intervals of a predetermined length of time. In this case, the sensor control unit 2 is preferably provided with a data storage means that is similar to the storage portion 43 of the data processing unit 4.

Next, an action of the sensor insertion method, the analysis method, and the sensor insertion device A1 of this embodiment will be described.

According to this embodiment, as shown in Fig. 2, when the unit fixing step is completed, the relative position between the sensor inserting unit 1 and the sensor control unit 2 in the sensor insertion allowing state is regulated. Subsequently, after the sensor inserting step shown in Fig. 4 is ended, the sensor control unit 2 is shifted from the sensor insertion allowing state to the sensor connecting state as shown in Fig. 6, and, thus, the connection between the sensor 10 and the sensor control unit 2 is completed. Accordingly, it is not necessary that the sensor control unit 2 whose position or movement direction is not regulated at all with respect to the inserted sensor 10 be connected to the sensor 10 by a user by touch or the like, and, thus, a situation in which the user feels pain is suppressed. Thus, according to this embodiment, the sensor 10 and the sensor control unit 2 can be more properly connected to each other.

In this embodiment, the operations shown in Figs. 1 and 2 among those in the unit fixing step can be performed, for example, on a table, before securing to the insertion target portion 81. Accordingly, the operation can be performed easily and reliably compared with the case of performing similar operations on the insertion target portion 81, which is part of the body. Accordingly, a situation can be avoided in which an unexpected trouble in the fixing operation inhibits the insertion of the sensor 10 or prevents the analysis to be properly performed.

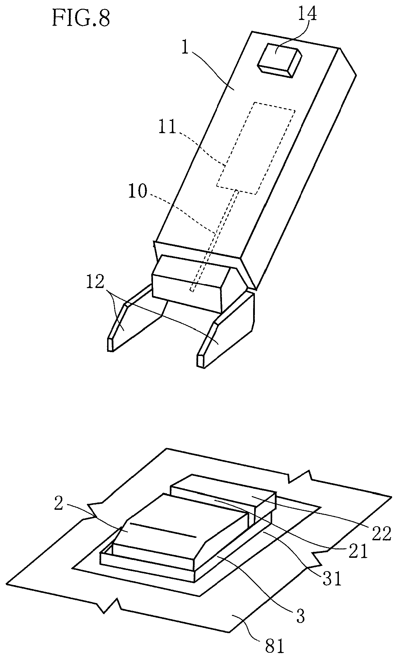

Furthermore, in this embodiment, when the sensor control unit 2 is shifted from the sensor insertion allowing state to the sensor connecting state as shown in Fig. 6, the sliding portion 22 is moved in a direction along the surface of the insertion target portion 81. Since the sensor 10 has been inserted in a direction inclined with respect to the surface of the insertion target portion 81, the movement direction of the sliding portion 22 also intersects the sensor 10. Accordingly, when moving the sliding portion 22, a situation can be avoided in which the sensor control unit 2 presses the sensor 10 more deeply into the insertion target portion 81. This feature is suited to prevent unnecessary pain of the user. Figs. 8 to 30 show other embodiments of the present disclosure.

In these drawings, the same or similar constituent elements to those in the foregoing embodiment are denoted by the same reference numerals as those in the embodiment.

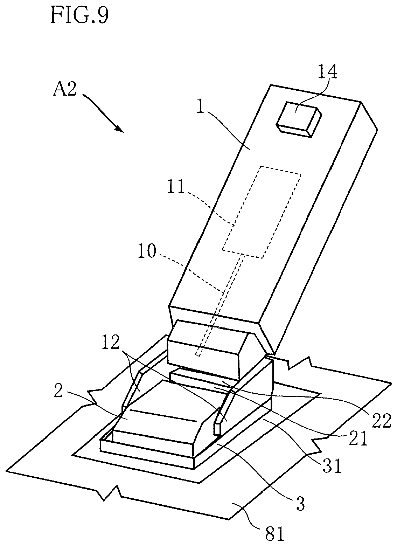

Figs. 8 and 9 show a sensor insertion method, an analysis method, and a sensor insertion device according to a second embodiment of the present disclosure. In this embodiment, the unit base 3 to which the sensor control unit 2 has been fixed in the unit fixing step shown in Fig. 8 is secured by the securing tape 31 to the insertion target portion 81. Next, the sensor inserting unit 1 is fixed to the unit base 3 secured to the insertion target portion 81, and, thus, the unit fixing step is completed as shown in Fig. 9, and a sensor insertion device A2 is completed. The operation shown in Fig. 8 may be performed such that, after the unit base 3 is secured to the insertion target portion 81, the sensor control unit 2 is fixed to the unit base 3. Subsequently, the sensor inserting step and the sensor connecting step described with reference to Figs. 4 to 7 are sequentially executed, and an analysis is performed.

Also with this embodiment, the sensor 10 and the sensor control unit 2 can be more properly connected to each other.

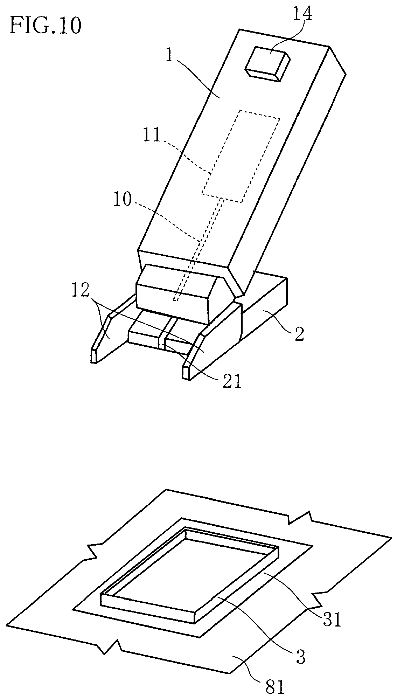

Figs . 10 to 13 show a sensor insertion method, an analysis method, and a sensor insertion device according to a third embodiment of the present disclosure. In this embodiment, as shown in Fig. 10, in the unit fixing step, the unit base 3 is secured by the securing tape 31 to the insertion target portion 81. Furthermore, the sensor control unit 2 is attached to the sensor inserting unit 1.

In this embodiment, the sensor control unit 2 is engaged with the two engagement portions 12 of the sensor inserting unit 1, so that the relative position with respect to the sensor inserting unit 1 is regulated. On the other hand, the unit base 3 does not positively regulate movement of the sensor control unit 2. In this embodiment, for example, the two engagement portions 12 are respectively provided with grooves (not shown) extending in the front-rear direction in the drawings . If part of the sensor control unit 2 is engaged with these grooves, the sensor control unit 2 is slidable in the front-rear direction in the drawings with respect to the sensor inserting unit 1, but the other movement thereof is inhibited. This state is an example of a state in which the relative position between the sensor inserting unit 1 and the sensor control unit 2 is regulated.

In this embodiment, as shown in Fig. 10, the whole of the sensor control unit 2 is withdrawn rearward in the drawing with respect to a passage through which the sensor 10 passes when the sensor 10 is inserted from the sensor inserting unit 1. This state is a sensor insertion allowing state in this embodiment. Furthermore, the sensor control unit 2 is provided with the through portion 21. The through portion 21 of this embodiment is in the shape of a slit that is open on the front side in the drawings, extends in the front-rear direction in the drawings, and passes through the sensor control unit 2 in the upper-lower direction in the drawings. If such a through portion 21 is provided, in the sensor inserting step of inserting the sensor 10, even when the sensor control unit 2 overlaps the passage through which the sensor 10 passes, an interfere between the sensor 10 and the sensor control unit 2 can be avoided by allowing the sensor 10 to pass through the through portion 21. Note that a configuration is also possible in which an interference between the sensor 10 and the sensor control unit 2 is avoided by causing the whole of the sensor control unit 2 to be clearly withdrawn.

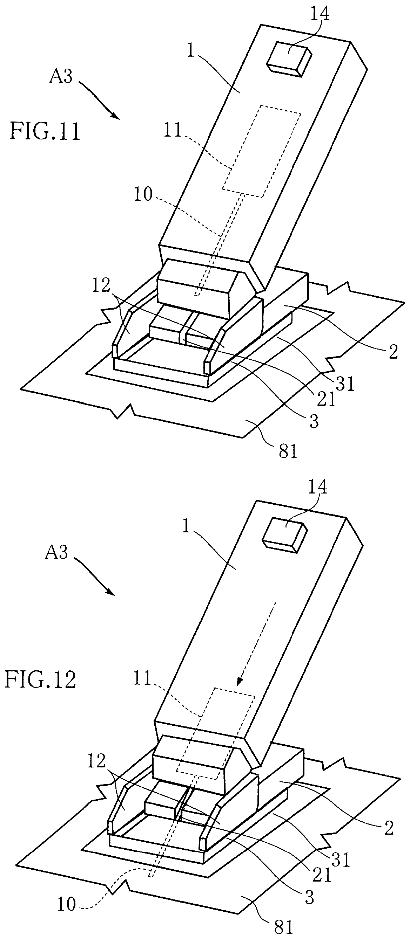

Next, as shown in Fig. 11, the sensor inserting unit 1 to which the sensor control unit 2 has been fixed is fixed to the unit base 3. Accordingly, the unit fixing step of this embodiment is completed, and a sensor insertion device A3 of this embodiment is completed.

Next, as shown in Fig. 12, the sensor 10 is inserted into the insertion target portion 81 by operating the inserting portion 11. Accordingly, the sensor inserting step of this embodiment is completed.

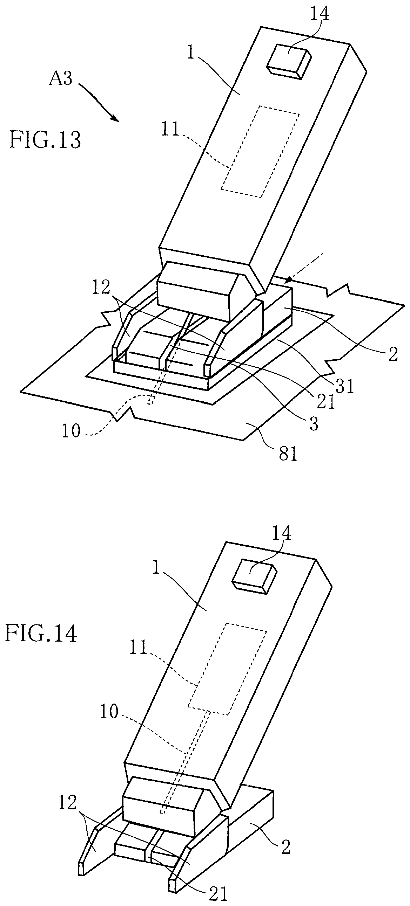

Next, as shown in Fig. 13, the sensor control unit 2 is slid forward in the drawing along the above-described grooves (not shown) of the engagement portions 12. Accordingly, the relative position of the sensor 10 changes to the deeper side in the through portion 21 of the sensor control unit 2. If the above-described plurality of connectors 27 are arranged at the deeper portion in the through portion 21, the plurality of electrode pads 101 of the sensor 10 and the plurality of connectors 27 are brought into contact with each other. Accordingly, the sensor connecting step in this embodiment is executed, the sensor control unit 2 is shifted to the sensor connecting state. At least after the sensor control unit 2 is shifted to the sensor connecting state, the sensor control unit 2 is directly fixed to the unit base 3. Accordingly, even after the sensor inserting unit 1 is removed, the sensor control unit 2 is fixed to the unit base 3, and the sensor connecting state is maintained.

Also with this embodiment, the sensor 10 and the sensor control unit 2 can be more properly connected to each other. In this embodiment, the sensor control unit 2 can be attached to the sensor inserting unit 1 on a table. This feature is advantageous in that, for example, the positioning between the insertion passage of the sensor 10 and the sensor control unit 2 can be more precisely performed.

Figs. 14 to 16 show a sensor insertion method, an analysis method, and a sensor insertion device according to a fourth embodiment of the present disclosure. In this embodiment, as shown in Fig. 14, the sensor control unit 2 is fixed to the two engagement portions 12 of the sensor inserting unit 1. The fixing of the sensor control unit 2 to the engagement portions 12 is as in the configuration in the above-described sensor insertion device A3.

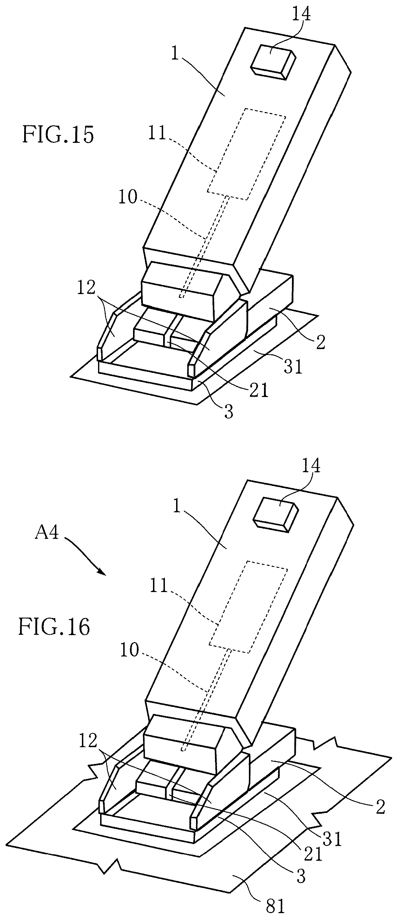

Next, as shown in Fig. 15, the engagement portions 12 of the sensor inserting unit 1 and the unit base 3 are fixed to each other. Note that, before or after the fixing, the securing tape 31 is attached to the unit base 3.

Next, as shown in Fig. 16, the unit base 3 is secured by the securing tape 31 to the insertion target portion 81. Accordingly, the unit fixing step of this embodiment is completed, and a sensor insertion device A4 of this embodiment is completed. Subsequently, the sensor inserting step described with reference to Fig. 12 and the sensor connecting step described with reference to Fig. 13 may be sequentially executed.

Also with this embodiment, the sensor 10 and the sensor control unit 2 can be more properly connected to each other. Furthermore, according to this embodiment, as shown in Fig. 15, the sensor inserting unit 1, the sensor control unit 2, and the unit base 3 can be completely fixed to each other on a table. Accordingly, it is sufficient to perform only the operation that secures the unit base 3 with the securing tape 31, near the insertion target portion 81. This feature is preferable for reducing the burden such as positioning performed by the user.

In the sensor insertion device A1 and the sensor insertion device A2, the sliding portion 22, which is part of the sensor control unit 2, is slid to shift the sensor control unit 2 from the sensor insertion allowing state to the sensor connecting state. On the other hand, in the sensor insertion device A3 and the sensor insertion device A4, the whole of the sensor control unit 2 is slid to shift the sensor control unit 2 from the sensor insertion allowing state to the sensor connecting state. These configurations can be selected as appropriate, and, in the sensor insertion device A1 and the sensor insertion device A2, the whole of the sensor control unit 2 may be slid. Furthermore, in the sensor insertion device A3 and the sensor insertion device A4, part of the sensor control unit 2 may be slid. Furthermore, when shifting the sensor control unit 2 from the sensor insertion allowing state to the sensor connecting state, the sensor inserting unit 1 may be fixed to the insertion target portion 81, or the sensor inserting unit 1 may be removed depending on the configuration of the sensor control unit 2.

Furthermore, although the configuration in which the whole or part of the sensor control unit 2 is moved along the surface of the insertion target portion 81 is preferable in that the above-described effects are achieved, the present disclosure is not limited thereto. The sensor control unit 2 may adopt various movement forms or deformation forms in order to be shifted from the sensor insertionallowing state to the sensor connecting state.

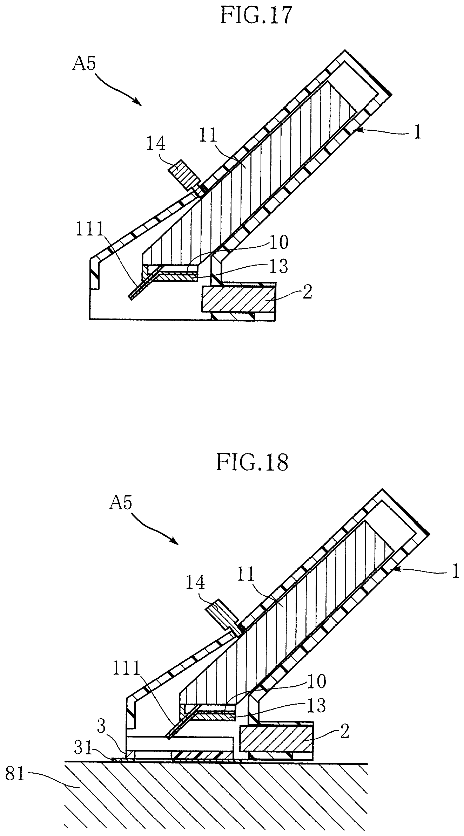

Figs. 17 to 22 show a sensor insertion method, an analysis method, and a sensor insertion device according to a fifth embodiment of the present disclosure. A sensor insertion device A5 of this embodiment includes a sensor holder 13 as shown in Fig. 17. The sensor holder 13 holds the sensor 10, and is held at the front end of the inserting portion 11 of the sensor inserting unit 1. The sensor 10 of this embodiment has a bent shape having a first half portion extending along the insertion direction and a second half portion extending along the left-right direction in the drawings along the sensor holder 13. The sensor control unit 2 is held in the rear portion of the sensor inserting unit 1. In this embodiment, a puncture assisting member 111 provided in the inserting portion 11 is shown in the drawings. Note that the sensor holder 13 is of a disposable type, that is, is intended to be disposed of after being used for a single time of sensor insertion and analysis.

After the sensor insertion device A5 is prepared, a unit fixing step shown in Fig. 18 is executed. In this embodiment, the sensor inserting unit 1 is fixed as in the foregoing embodiments to the unit base 3 secured by the securing tape 31 to the insertion target portion 81.

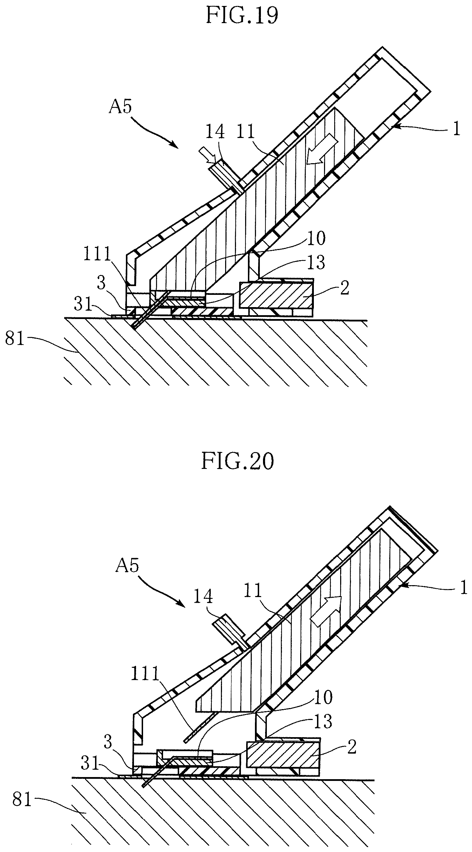

Next, a sensor inserting step shown in Fig. 19 is executed. As shown in this drawing, for example, if the user presses down the operation portion 14, the inserting portion 11 is advanced obliquely downward inside the sensor inserting unit 1. At that time, the puncture assisting member 111 and the sensor holder 13 held on the inserting portion 11 and the sensor 10 held by the sensor holder 13 are altogether moved obliquely downward. Thus, the tip of the puncture assisting member 111 and the tip of the sensor 10 are inserted into the insertion target portion 81. At the time of this insertion, mainly the puncture assisting member 111 exhibits the puncture function, and the sensor 10 is inserted into the insertion target portion 81 following the puncture assisting member 111. In this sensor inserting step, for example, part of the sensor holder 13 is engaged with the unit base 3, so that the sensor holder 13 is attached to the unit base 3.

After the puncture assisting member 111 and the sensor 10 are inserted, as shown in Fig. 20, the inserting portion 11 is immediately returned obliquely upward. At that time, the holding of the sensor holder 13 by the inserting portion 11 is canceled, and only the puncture assisting member 111 is removed by the inserting portion 11 from the insertion target portion 81.

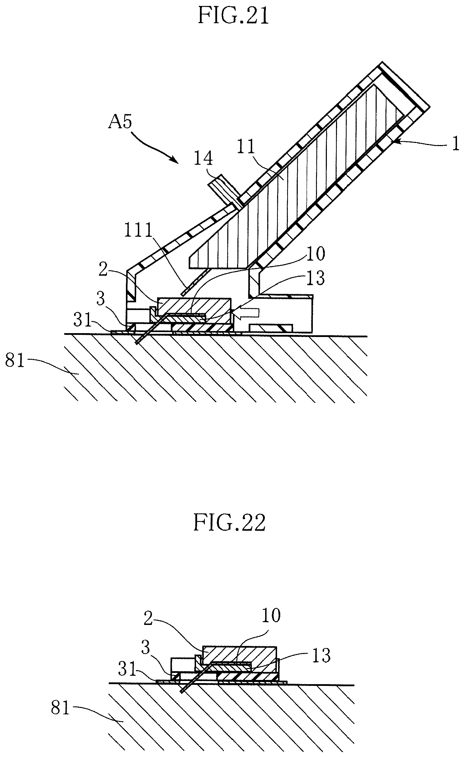

Next, a sensor connecting step shown in Fig. 21 is performed. In this embodiment, the sensor control unit 2 held in the rear portion of the sensor inserting unit 1 is slid to the left in the drawing, for example, along grooves (not shown) or the like of the sensor inserting unit 1, so that the sensor control unit 2 and the sensor holder 13 are brought into contact with each other. At that time, electrode pads (not shown) of the sensor 10 and connectors (not shown) of the sensor control unit 2 are connected to each other. The grooves or the like of the sensor inserting unit 1 functions as a guide during movement of the sensor control unit 2, and a situation in which the user feels pain is suppressed by preventing excessive force from being applied to the sensor 10 that has been inserted under the skin.

Next, the sensor inserting unit 1 is removed from the insertion target portion 81, for example, by canceling the engagement between the sensor inserting unit 1 and the unit base 3. Accordingly, the sensor insertion method of this embodiment is completed as shown in Fig. 22. Subsequently, an analysis using the sensor 10 step is executed as in the foregoing embodiments.

Also with this embodiment, the sensor 10 and the sensor control unit 2 can be more properly connected to each other. Furthermore, as seen from this embodiment, the configuration of the sensor 10 is not limited to those in which the entire sensor 10 extends in a straight shape, and may have various shapes such as a bent shape as long as it allows insertion into the insertion target portion 81. Furthermore, since the electrode pads of the sensor 10 and the connectors of the sensor control unit 2 are connected to each other while the sensor 10 is held in one piece with the sensor holder 13, a situation can be avoided in which wobbling or the like of the flexible sensor 10 makes it difficult to be connected to the sensor control unit 2.

Above, embodiments were described in which only the sensor control unit 2 is moved to shift the control unit 2 from the sensor insertion allowing state to the sensor connecting state in which electrical conduction is established between the sensor 10 and the sensor control unit 2, but the present disclosure is not limited thereto. A configuration is also possible in which the sensor control unit 2 is moved together with the sensor inserting unit 1 holding the sensor control unit 2 to establish electrical conduction between the sensor 10 and the sensor control unit 2. In this case, the sensor control unit 2 is fixed to the sensor holder 13 when brought into contact with the sensor holder 13, but the sensor inserting unit 1 may be moved to a position outside the unit base 3 and be released from the unit base 3 and the insertion target portion 81.

Above, embodiments were described in which part or the whole of the sensor control unit 2 is moved in a direction along the surface of the insertion target portion 81 to shift the control unit 2 from the sensor insertion allowing state to the sensor connecting state, but the method for changing the state of the sensor control unit 2 is not limited to this. For example, part or the whole of the sensor control unit 2 may be moved not in the direction along the surface of the insertion target portion 81 but in an upper direction or an obliquely upper direction.

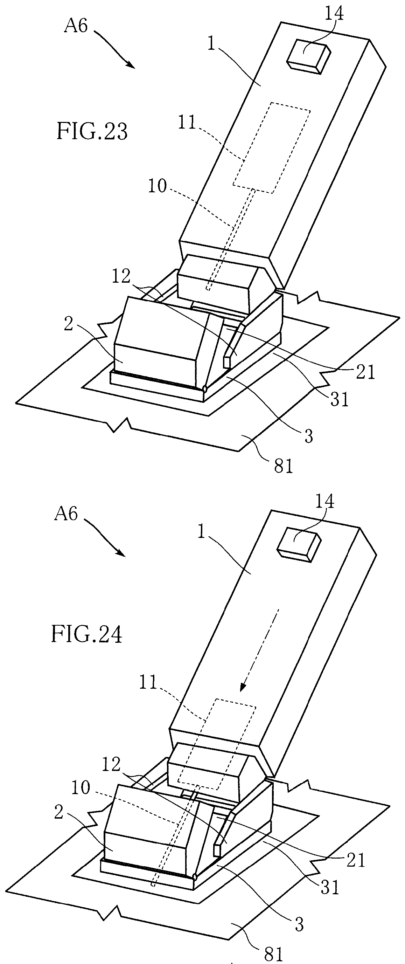

Figs. 23 to 25 show a sensor insertion method, an analysis method, and a sensor insertion device according to a sixth embodiment of the present disclosure. As shown in Fig. 23, a sensor insertion device A6 of this embodiment has a conf iguration similar to that of the above-described sensor insertion device A1, except for the configuration of the sensor control unit 2. The relative position of the sensor control unit 2 with respect to the sensor inserting unit 1 is fixed via the unit base 3. In this embodiment, the sensor control unit 2 is turnably supported by the unit base 3. With this turning, the sensor control unit 2 is shifted between the sensor insertion allowing state and the sensor connecting state in the present disclosure. In Fig. 23, the sensor control unit 2 is in the sensor insertion allowing state.

In the sensor inserting step shown in Fig. 24, the sensor 10 is inserted by the inserting portion 11 of the sensor inserting unit 1 through the through portion 21 formed on the deeper side in the drawing in the sensor control unit 2 into the insertion target portion 81. Then, the sensor inserting unit 1 is released from the unit base 3. Accordingly, the unit base 3 and the sensor control unit 2 are fixed via the securing tape 31 to the insertion target portion 81, and the state is realized in which the sensor 10 is inserted.

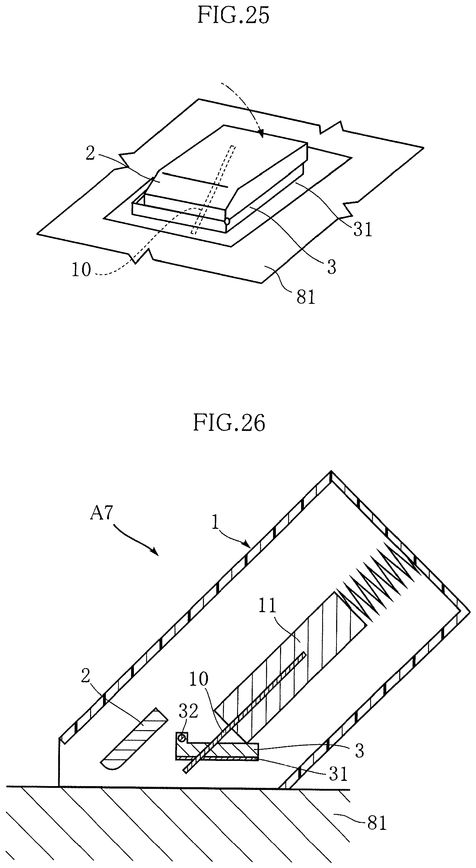

Next, in the sensor connecting step shown in Fig. 25, the sensor control unit 2 is turned relative to the unit base 3, so that the sensor control unit 2 and the sensor 10 are electrically connected to each other. Accordingly, the sensor control unit 2 is in the sensor connecting state in the present disclosure.

Also with this embodiment, the sensor 10 and the sensor control unit 2 can be more properly connected to each other.

Figs . 26 to 30 show a sensor insertion method, an analysis method, and a sensor insertion device according to a seventh embodiment of the present disclosure. As shown in Fig. 26, a sensor insertion device A7 of this embodiment is configured such that the sensor inserting unit 1 has an opening on the lower side in the drawing. The unit base 3 is disposed inside the sensor inserting unit 1. Furthermore, the sensor control unit 2 is inserted from the opening of the sensor inserting unit 1 and is fixed inside the sensor inserting unit 1. The inserting portion 11 can be advanced and returned in directions obliquely connecting the upper right and the lower left in the drawing, by a mechanism such as a spring.

The lower face of the unit base 3 is provided with the securing tape 31. Furthermore, the unit base 3 has an engagement portion 32. The engagement portion 32 is a portion that is engaged with the sensor control unit 2 in a step, which will be described later, so as to connect the unit base 3 and the sensor control unit 2 in one piece. As shown in Fig. 26, the opening of the sensor inserting unit 1 is brought into contact with the insertion target portion 81.

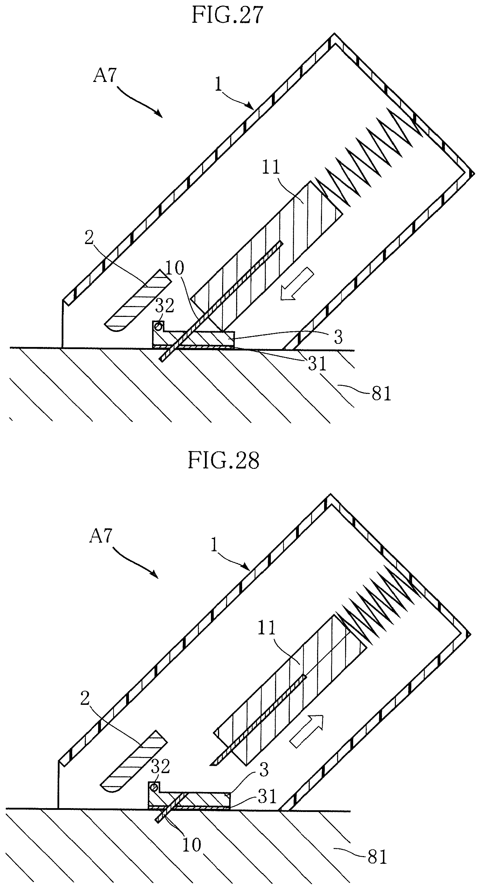

Next, for example, if the user presses down the operation portion 14, as shown in Fig. 27, the inserting portion 11 is obliquely advanced toward the lower left in the drawing. At that time, the electrochemical sensor 10 and the unit base 3 are advanced together with the inserting portion 11. Thus, the tip of the electrochemical sensor 10 is advanced into the insertion target portion 81. If the securing tape 31 of the unit base 3 is brought into contact with the insertion target portion 81, the unit base 3 is secured to the insertion target portion 81 due to a securing force of the securing tape 31. These steps are the sensor inserting step and the unit fixing step in this embodiment.

Next, as shown in Fig. 28, the inserting portion 11 is obliquely returned toward the upper right in the drawing. The unit base 3 is removed from the sensor inserting unit 1. At that time, a desired portion of the electrochemical sensor 10 is left in the insertion target portion 81. As a result, a desired portion of the electrochemical sensor 10 is combined in the unit base 3 secured to the insertion target portion 81.

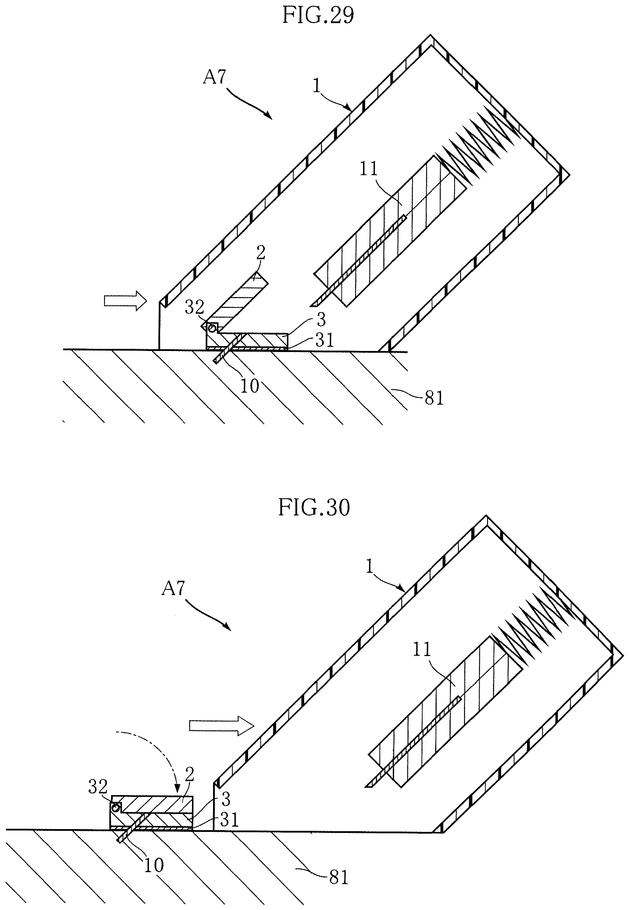

Next, as shown in Fig. 29 and according to the invention, the sensor inserting unit 1 is moved to the right in the drawing. At that time, the inserting portion 11 and the sensor control unit 2 are moved to the right in the drawing together with the sensor inserting unit 1. Accordingly, the sensor control unit 2 and the engagement portion 32 of the unit base 3 are engaged with each other. Furthermore, at that time, the sensor control unit 2 comes off the sensor inserting unit 1. The engagement portion 32 is engaged with the sensor control unit 2, so that the sensor control unit 2 is turnably supported by the unit base 3. Note that these specific configurations of the engagement portion 32 can be variously set. Furthermore, a portion having the function of the engagement portion 32 may be provided on the sensor control unit 2 or may be provided on both the sensor control unit 2 and the unit base 3.

Next, according to the invention, the sensor inserting unit 1 is further moved to the right in the drawing. In the state shown in Fig. 29 and thereafter, the sensor control unit 2 is engaged with the unit base 3. Accordingly, only the inserting portion 11 is moved to the right in the drawing together with the sensor inserting unit 1, and a desired portion of the electrochemical sensor 10, the sensor control unit 2, and the unit base 3 are not moved together with the sensor inserting unit 1. Then, the sensor control unit 2 obliquely standing toward the upper right is turned as shown in Fig. 30 by the front end of the sensor inserting unit 1 that is being moved to the right in the drawing. When the front end of the sensor inserting unit 1 completely passes over the sensor control unit 2, the sensor control unit 2 is, for example, turned to the horizontal orientation and is brought into contact with the unit base 3. With this contact, the electrochemical sensor 10 is electrically connected to the sensor control unit 2. This step is the sensor connecting step in this embodiment. After the sensor inserting unit 1 is removed from the insertion target portion, predetermined analysis processing is performed. Note that the front end of the sensor inserting unit 1 may be provided with a roller (not shown) so that the sensor inserting unit 1 can properly turn the sensor control unit 2 or press the sensor control unit 2 against the unit base 3.

Also with this embodiment, the sensor 10 and the sensor control unit 2 can be more properly connected to each other. After the sensor inserting step shown in Fig. 27 and the returning of the inserting portion 11 shown in Fig. 28 are performed in this order, as shown in Figs. 29 and 30, the sensor inserting unit 1 is moved along the insertion target portion 81 to bring the sensor control unit 2 into contact with the unit base 3 and to connect the electrochemical sensor 10 to the sensor control unit 2, at a time. This feature is suited to efficiently perform the sensor insertion method. The user can bring the sensor control unit 2 into contact with the unit base 3 and execute the sensor connecting step, merely by performing an operation that touches the sensor inserting unit 1, without touching the sensor control unit 2 and the unit base 3. This feature is preferable for an improvement of the hygienic state of the sensor control unit 2 and the unit base 3, for example.

It should be noted that the sensor insertion method, the analysis method, and the sensor insertion device according to the present invention are not limited to the foregoing embodiments. The specific configuration of the sensor insertion method, the analysis method, and the sensor insertion device according to the present invention can be variously designed and modified. The scope of protection is defined exclusively by the appended claims.

## Claims

1. A non-surgical sensor insertion method for inserting a sensor (10) into a user's body, the sensor being a continuous glucose monitoring sensor (CGMS), the method comprising:
an attaching step of attaching the sensor to a unit base (3);
a unit fixing step of regulating a relative position between a sensor control unit (2) and an inserting unit (1), the sensor control unit (2) being fixed inside the inserting unit (1) and being in a sensor insertion allowing state, the inserting unit (1) containing the sensor (10) therein, and of fixing the sensor control unit (2) and the inserting unit (1) to an insertion target portion (81) of the user;
a sensor inserting step of causing the inserting unit (1) to insert the sensor (10), which is attached to the unit base, into the insertion target portion (81);
a fixing step of fixing the unit base (3) and thereby also the sensor, which is attached to the unit base (3), in a state that the sensor is inserted into the insertion target portion of the user; and
a sensor connecting step of shifting the sensor control unit (2) from the sensor insertion allowing state to a sensor connecting state, thereby establishing electrical conduction between the sensor (10) in the insertion target portion (81) and the sensor control unit (2),
wherein, in the sensor connecting step, the sensor (10) and the sensor control unit (2) are electrically connected to each other by rotating of the sensor control unit (2), via an engagement portion (32) configured to allow for rotatable engagement of sensor control unit (2) and the unit base (3), the engagement portion (32) provided either on the sensor control unit (2), or the unit base (3), or on both;
wherein the sensor control unit (2) rotatably engages with the unit base (3) by moving of the inserting unit (1) along the insertion target portion (81).

2. The sensor insertion method according to any one of the preceding claims, wherein, in the unit fixing step, after the relative position between the sensor control unit (2) and the inserting unit (1) is regulated, the sensor control unit (2) and the inserting unit (1) are fixed to the insertion target portion (81).

3. The sensor insertion method according to any one of claims 1 or 2, further comprising a unit releasing step of releasing the inserting unit (1) from the insertion target portion (81) after the sensor connecting step.

4. The sensor insertion method according to any of claims 1-3, further comprising an analysis step of performing an analysis using the sensor (10) after the sensor insertion method according to any one of claims 1 to 3 is performed.

5. A sensor insertion device for inserting a sensor (10) for analysis into a user's body, the device comprising:
an inserting unit (1) that inserts the sensor (10) into an insertion target portion of the user;
a unit base that the sensor is attached and which is installed inside the inserting unit (1); and
a sensor control unit (2) regulated in relative position with respect to the inserting unit (1) and being fixed inside the inserting unit (1),
an engagement portion (32) configured to allow for rotatable engagement of sensor control unit (2) and the unit base (3), the engagement portion (32) provided either on the sensor control unit (2), or the unit base (3), or on both;
wherein the sensor control unit (2) is configured to selectively take a sensor insertion allowing state allowing the insert of the sensor (10) while the inserting unit (1) is fixed to the insertion target portion (81), and a sensor connecting state in which the sensor control unit (2) is electrically connected to the sensor (10) inserted into the insertion target portion (81), wherein the inserting unit (1) causes the unit base and the sensor (10) attached to the unit base to move, inserts the sensor (10) into the insertion target portion (81), and fixes the unit base on the insertion target portion (81), and
wherein the unit base fixes the sensor (10) in a state that the sensor is inserted into the insertion target portion (81) of the user,
wherein the sensor control unit (2) is configured to establish an electrical conduction between the sensor (10) and the sensor control unit (2) by rotation of the sensor control unit (2), wherein the sensor control unit (2) is configured to rotatably engage with the unit base (3), via the engagement portion (32), by moving of the inserting unit (2) along the insertion target portion (81).

6. The sensor insertion device according to claim 5, wherein the sensor control unit (2 is shifted from the sensor insertion allowing state to the sensor connecting state when the sensor control unit (2) is moved in a direction along a surface of the insertion target portion (81).

## Patentansprüche

1. Nicht-chirurgisches Sensoreinführungsverfahren zum Einführen eines Sensors (10) in den Körper eines Benutzers,
wobei der Sensor ein Sensor zur kontinuierlichen Glukoseüberwachung (CGMS) ist, wobei das Verfahren Folgendes umfasst:
einen Anbringungsschritt zum Anbringen des Sensors an einer Einheitsbasis (3);
einen Einheitsbefestigungsschritt des Einstellens einer relativen Position zwischen einer Sensorsteuereinheit (2) und einer Einführungseinheit (1), wobei die Sensorsteuereinheit (2) innerhalb der Einführungseinheit (1) befestigt ist und sich in einem Sensoreinführungserlaubniszustand befindet, wobei die Einführungseinheit (1) den Sensor (10) darin enthält, und des Befestigens der Sensorsteuereinheit (2) und der Einführungseinheit (1) an einem Einführungszielabschnitt (81) des Benutzers;
einen Sensoreinführungsschritt des Veranlassens, dass die Einführungseinheit (1) den Sensor (10), der an der Einheitsbasis angebracht ist, in den Einführungszielabschnitt (81) einführt;
einen Befestigungsschritt des Befestigens der Einheitsbasis (3) und damit auch des Sensors, der an der Einheitsbasis (3) angebracht ist, in einem Zustand, in dem der Sensor in den Einführungszielabschnitt des Benutzers eingeführt ist; und
einen Sensorverbindungsschritt des Umschaltens der Sensorsteuereinheit (2) von dem Sensoreinführungserlaubniszustand in einen Sensorverbindungszustand, wodurch eine elektrische Leitung zwischen dem Sensor (10) in dem Einführungszielabschnitt (81) und der Sensorsteuereinheit (2) hergestellt wird,
wobei in dem Sensorverbindungsschritt der Sensor (10) und die Sensorsteuereinheit (2) durch Drehen der Sensorsteuereinheit (2) über einen Eingriffsabschnitt (32) elektrisch miteinander verbunden werden, wobei der Eingriffsabschnitt so ausgebildet ist, dass er einen drehbaren Eingriff zwischen der Sensorsteuereinheit (2) und der Einheitsbasis (3) erlaubt, wobei der Eingriffsabschnitt (32) entweder an der Sensorsteuereinheit (2) oder der Einheitsbasis (3) oder an beiden vorgesehen ist;
wobei die Sensorsteuereinheit (2) drehbar mit der Einheitsbasis (3) in Eingriff steht, indem die Einführungseinheit (1) entlang des Einführungszielabschnitts (81) bewegt wird.

2. Sensoreinführungsverfahren nach einem der vorstehenden Ansprüche, wobei in dem Einheitsbefestigungsschritt, nachdem die relative Position zwischen der Sensorsteuereinheit (2) und der Einführungseinheit (1) eingestellt ist, die Sensorsteuereinheit (2) und die Einführungseinheit (1) an dem Einführungszielabschnitt (81) befestigt werden.

3. Sensoreinführungsverfahren nach einem der Ansprüche 1 oder 2, das weiter einen Einheitsfreigabeschritt des Freigebens der Einführungseinheit (1) von dem Einführungszielabschnitt (81) nach dem Sensorverbindungsschritt umfasst.

4. Sensoreinführungsverfahren nach einem der Ansprüche 1 bis 3, weiter umfassend einen Analyseschritt des Durchführens einer Analyse unter Verwendung des Sensors (10), nachdem das Sensoreinführungsverfahren nach einem der Ansprüche 1 bis 3 durchgeführt wurde.

5. Sensoreinführungsvorrichtung zum Einführen eines Sensors (10) zur Analyse in den Körper eines Benutzers, wobei die Vorrichtung Folgendes umfasst:
eine Einführungseinheit (1), die den Sensor (10) in einen Einführungszielabschnitt des Benutzers einführt;
eine Einheitsbasis, an der der Sensor angebracht ist und die im Inneren der Einführungseinheit (1) installiert ist; und
eine Sensorsteuereinheit (2), die in Bezug auf die Einführungseinheit (1) in ihrer relativen Position eingestellt und im Inneren der Einführungseinheit (1) befestigt ist, einen Eingriffsabschnitt (32), der so ausgebildet ist, dass er einen drehbaren Eingriff zwischen der Sensorsteuereinheit (2) und der Einheitsbasis (3) erlaubt, wobei der Eingriffsabschnitt (32) entweder an der Sensorsteuereinheit (2) oder der Einheitsbasis (3) oder an beiden vorgesehen ist;
wobei die Sensorsteuereinheit (2) so ausgebildet ist, dass sie selektiv einen Sensoreinführungserlaubniszustand einnimmt, der das Einführen des Sensors (10) erlaubt, während die Einführungseinheit (1) an dem Einführungszielabschnitt (81) befestigt ist, und einen Sensorverbindungszustand, in dem die Sensorsteuereinheit (2) elektrisch mit dem in den Einführungszielabschnitt (81) eingeführten Sensor (10) verbunden ist, wobei die Einführungseinheit (1) veranlasst, dass sich die Einheitsbasis und der an der Einheitsbasis angebrachte Sensor (10) bewegen, den Sensor (10) in den Einführungszielabschnitt (81) einführt und die Einheitsbasis an dem Einführungszielabschnitt (81) befestigt, und
wobei die Einheitsbasis den Sensor (10) in einem Zustand befestigt, in dem der Sensor in den Einführungszielabschnitt (81) des Benutzers eingeführt ist,
wobei die Sensorsteuereinheit (2) so ausgebildet ist, dass sie eine elektrische Leitung zwischen dem Sensor (10) und der Sensorsteuereinheit (2) durch Drehung der Sensorsteuereinheit (2) herstellt, wobei die Sensorsteuereinheit (2) so ausgebildet ist, dass sie über den Eingriffsabschnitt (32) drehbar mit der Einheitsbasis (3) in Eingriff steht, durch Bewegen der Einführungseinheit (2) entlang des Einführungszielabschnitts (81).

6. Sensoreinführungsvorrichtung nach Anspruch 5, wobei die Sensorsteuereinheit (2) von dem Sensoreinführungserlaubniszustand in den Sensorverbindungszustand umgeschaltet wird, wenn die Sensorsteuereinheit (2) in einer Richtung entlang einer Oberfläche des Einführungszielabschnitts (81) bewegt wird.

## Revendications

1. Procédé non chirurgical d'insertion de capteur pour insérer un capteur (10) dans le corps d'un utilisateur, le capteur étant un capteur de surveillance de glucose en continu (CGMS), le procédé comprenant :
une étape de fixation pour attacher le capteur à une base d'unité (3) ;
une étape de fixation d'unité consistant à régler une position relative entre une unité de commande de capteur (2) et une unité d'insertion (1), l'unité de commande de capteur (2) étant fixée à l'intérieur de l'unité d'insertion (1) et se trouvant dans un état d'autorisation d'insertion de capteur, l'unité d'insertion (1) contenant le capteur (10) en elle, et à fixer l'unité de commande de capteur (2) et l'unité d'insertion (1) à une partie cible d'insertion (81) de l'utilisateur ;
une étape d'insertion de capteur consistant à amener l'unité d'insertion (1) à insérer le capteur (10), qui est fixé à la base d'unité, dans la partie cible d'insertion (81) ;
une étape de fixation consistant à fixer la base d'unité (3) et donc aussi le capteur, qui est attaché à la base d'unité (3), dans un état dans lequel le capteur est inséré dans la partie cible d'insertion de l'utilisateur ; et
une étape de connexion de capteur consistant à faire passer l'unité de commande de capteur (2) de l'état d'autorisation d'insertion de capteur dans un état de connexion de capteur, établissant de ce fait une conduction électrique entre le capteur (10) dans la partie cible d'insertion (81) et l'unité de commande de capteur (2),
dans lequel, lors de l'étape de connexion de capteur, le capteur (10) et l'unité de commande de capteur (2) sont connectés électriquement l'un à l'autre par une rotation de l'unité de commande de capteur (2), par l'intermédiaire d'une partie d'engagement (32) configurée de manière à permettre un engagement rotatif de l'unité de commande de capteur (2) et de la base d'unité (3), la partie d'engagement (32) étant prévue soit sur l'unité de commande de capteur (2), soit sur la base d'unité (3), soit sur les deux ;
dans lequel l'unité de commande de capteur (2) vient en engagement de façon rotative avec la base d'unité (3) en déplaçant l'unité d'insertion (1) le long de la partie cible d'insertion (81).

2. Procédé d'insertion de capteur selon l'une quelconque des revendications précédentes, dans lequel, lors de l'étape de fixation d'unité, après que la position relative entre l'unité de commande de capteur (2) et l'unité d'insertion (1) ait été réglée, l'unité de commande de capteur (2) et l'unité d'insertion (1) sont fixées à la partie cible d'insertion (81).

3. Procédé d'insertion de capteur selon l'une quelconque des revendications 1 ou 2, comprenant en outre une étape de libération d'unité consistant à libérer l'unité d'insertion (1) de la partie cible d'insertion (81) après l'étape de connexion de capteur.

4. Procédé d'insertion de capteur selon l'une quelconque des revendications 1 à 3, comprenant en outre une étape d'analyse consistant à effectuer une analyse en utilisant le capteur (10) après l'exécution du procédé d'insertion de capteur selon l'une quelconque des revendications 1 à 3.

5. Dispositif d'insertion de capteur pour insérer un capteur (10) pour analyse dans le corps d'un utilisateur, le dispositif comprenant :
une unité d'insertion (1) qui insère le capteur (10) dans une partie cible d'insertion de l'utilisateur ;
une base d'unité à laquelle le capteur est attaché qui est installée à l'intérieur de l'unité d'insertion (1) ; et
une unité de commande de capteur (2) qui est réglée dans une position relative par rapport à l'unité d'insertion (1) et qui est fixée à l'intérieur de l'unité d'insertion (1), une partie d'engagement (32) qui est configurée de manière à permettre un engagement rotatif de l'unité de commande de capteur (2) et de la base d'unité (3), la partie d'engagement (32) étant prévue soit sur l'unité de commande de capteur (2), soit sur la base d'unité (3), soit sur les deux ;
dans lequel l'unité de commande de capteur (2) est configurée de manière à adopter sélectivement un état d'autorisation d'insertion de capteur qui autorise l'insertion du capteur (10) alors que l'unité d'insertion (1) est fixée à la partie cible d'insertion (81),
et un état de connexion de capteur dans lequel l'unité de commande de capteur (2) est connectée électriquement au capteur (10) inséré dans la partie cible d'insertion (81), dans lequel l'unité d'insertion (1) amène la base d'unité et le capteur (10) attaché à la base d'unité à se déplacer, insère le capteur (10) dans la partie cible d'insertion (81) et fixe la base d'unité sur la partie cible d'insertion (81), et
dans lequel la base d'unité fixe le capteur (10) dans un état dans lequel le capteur est inséré dans la partie cible d'insertion (81) de l'utilisateur,
dans lequel l'unité de commande de capteur (2) est configurée de manière à établir une conduction électrique entre le capteur (10) et l'unité de commande de capteur (2) par une rotation de l'unité de commande de capteur (2),
dans lequel l'unité de commande de capteur (2) est configurée de manière à venir en engagement de façon rotative avec la base d'unité (3), par l'intermédiaire de la partie d'engagement (32),
en déplaçant l'unité d'insertion (2) le long de la partie cible d'insertion (81).

6. Dispositif d'insertion de capteur selon la revendication 5,
dans lequel l'unité de commande de capteur (2) passe de l'état d'autorisation d'insertion de capteur à l'état de connexion de capteur lorsque l'unité de commande de capteur (2) est déplacée dans une direction le long d'une surface de la partie cible d'insertion (81).
